# EUROPEAN PATENT APPLICATION

(11) **EP 3 520 687 A1**
(43) Date of publication of application: **07.08.2019**
(21) Application number: 18155081.5
(22) Date of filing: 05.02.2018
(51) Int. Cl.: A61B 5/00, A61B 5/055

(54) **CAMERA-BASED COIL CONFIGURATION IN MRI**

(71) Applicant: Koninklijke Philips N.V., 5656 AE Eindhoven (NL)
(72) Inventor: KRUEGER, Sascha, 5656 AE Eindhoven (NL); SENEGAS, Julien, 5656 AE Eindhoven (NL); KOKEN, Peter, 5656 AE Eindhoven (NL); VAN DEN BRINK, Johan Samuel, 5656 AE Eindhoven (NL)
(74) Representative: de Haan, Poul Erik

(57) **Abstract**

The invention provides for a medical apparatus (100) for imaging a target volume (130) of a subject (106), comprising:
- a coil arrangement (109) being configured to be disposed on the subject (106) and to receive signals from the subject;
- a scanning sensor (110);
- a scanning imaging system (121) being configured for acquiring image data from a predefined imaging zone (124) using the coil arrangement (109);
- a memory (120) containing machine executable instructions (140);
- a processor (116), wherein execution of the machine executable instructions (140) cause the processor (116) to:
- acquire with the sensor (110) sensor data that can be used to generate a of the subject (106) and the coil arrangement;
- identify coil image data representing the coil (109) in the sensor data (142);
- determine values of one or more configuration parameters of the coil (109) using the identified coil image data;
- determine using the parameter values and the sensor data (142) a scan position indicative of a position of the target volume (130) within the imaging zone (124); and
- control the scanning imaging system (121) for acquiring the image data from the target volume (130) in accordance with the determined scan position.

## Description

### FIELD OF THE INVENTION

The invention relates to medical imaging modalities such as magnetic resonance imaging; in particular to the positioning of a subject for the imaging of the subject.

### BACKGROUND OF THE INVENTION

Planning of the scan position for scanning purpose such as for magnetic resonance imaging (MRI) is done manually by an operator using a light visor. This costs time and is difficult even for experienced operators since the patient is already covered by coils, blankets, accessories etc., has safety issues due to the use of a laser, and is sometimes a cause of patient discomfort because of repeated table motion back and forth. Hence there is a need to improve this step of patient scanning.

### SUMMARY OF THE INVENTION

The invention provides for a medical apparatus, a computer program product and a method in the independent claims. Embodiments are given in the dependent claims.

The present disclosure uses depth cameras to recognize surface coils automatically in the scene and to determine their type and position. The calculated coil position and coil type can then be used as basis to automatically determine the target scan position. Moreover, coils detected on the image that are not properly connected to the scanning imaging system (e.g. an MR system) are flagged for safety check. Potential collisions with the bore can then be determined and identified regions are graphically marked for the user of the scanning imaging system to resolve the issue. The patient table travel can be checked against installed equipment and available cable lengths (e.g. IV infusions) and respective warnings are provided if e.g. typical IV tubing or cable lengths can be exceeded during patient table travel to a scan position.

In one aspect, the invention relates to a medical apparatus for imaging a target volume of a subject. The medical apparatus comprises: a coil arrangement being configured to be disposed on the subject and to receive signals from the subject; a scanning sensor; a scanning imaging system being configured for acquiring image data from a predefined imaging zone using the coil arrangement; a memory containing machine executable instructions; a processor, wherein execution of the machine executable instructions cause the processor to: acquire with the scanning sensor, sensor data that can be used to generate position attributes of the subject and the coil arrangement; identify coil image data representing the coil in the sensor data; determine values of one or more configuration parameters of the coil arrangement using the identified coil image data; determine using at least one of the parameter values and the sensor data a scan position indicative of a position of the target volume within the imaging zone; and control the scanning imaging system for acquiring the image data from the target volume in accordance with the determined scan position. For example, the sensor data can be used to generate a three-dimensional, 3D, representation indicative of the subject and/or the coil arrangement.

The coil arrangement comprises one or more coils. For example, the coil arrangement may be an anterior coil or a neurovascular and anterior coil or two anterior coils.

The configuration parameters of the coil arrangement may for example comprise the position of each coil of the coil arrangement. The scan position of the target volume maybe determined using the position of the coil that is associated with the target volume. For example, if multiple surveys are defined in an ExamCard of the medical apparatus, a first survey (e.g. the survey that corresponds to the target volume) may be inferred by the relative position of the surveys contained in the ExamCard. E.g the first survey in the ExamCard is further towards head than all the others, then the coil of the coil arrangement that is located furthest towards the head may be used to determine the scan position for the first survey.

The execution of the machine executable instructions may for example automatically be performed upon receiving a request e.g. from a user, or upon detecting the presence of the subject. For example, the presence of the subject may be detected by the scanning sensor. The scanning sensor may comprise at least one camera e.g. an RGB or depth camera.

The scanning sensor comprises an image sensor that is configured to acquire the sensor data. The sensor data may be processed to generate a 3D representation of the subject and the coil arrangement. The sensor data may be processed to generate position attributes of the subject and the coil arrangement, wherein the 3D representation is defined by the position attributes. The sensor data may for example refer to monochrome or RGB image pixel data or data where the positions of pixels or voxels of a scanned object are defined by the three-dimensional coordinates. The sensor data is, for example, image data composed of three-dimensionally arranged voxels. The sensor data may be referred to as volumetric data, voxel data, pixel data or the like. Any such sensor data may be used to determine the position of the subject or object in 3D space. The position of the subject and/or the parameter values may be used to determine the scan position.

The control of the scanning imaging system for acquiring the image data from the target volume in accordance with the determined scan position may comprise moving the subject into the direction of the imaging zone such that the scan position is achieved.

The coil may for example be a surface or local coil and may comprise at least one of a head coil, torso coil, back coil, cardiac coil, knee coil, and leg coil.

The scanning sensor may comprise an RGB or depth camera. This may enable to accurately and automatically recognize coils in the scene and to determine their position. Coil position is then used as basis to automatically determine the target san position.

The present disclosure may significantly speed up the scanning workflow and reduce errors in placing and connecting coils for scanning purpose. Another advantage may be that the scanning is more accurate because of the precise determination of the scanning position in 3D. The present disclosure enables for example to derive the position of the coil and the subject to be examined on a patient carrier without the need of additional markers. Also the type of the detected coil may be identified with its position/orientation of the subject's body.

The position attributes of the subject and coil arrangement define a three-dimensional, 3D, representation of the subject and the coil arrangement.

According to one embodiment, the determining of the configuration parameter values comprises comparing the identified data with reference data descriptive of the coil arrangement. The sensor data may comprise pixels, wherein each pixel is associated with an intensity and/or position value of the pixel. The identified coil image data may comprise a group of pixels that are indicative of the coil. The comparison may for example be performed in order to find a group of pixels in the reference data that matches better (closest to) the group of pixels of the identified image data.

The reference data may for example comprise multiple template data descriptive of the coil in respective different configurations of the coil. Each of the template data may be associated with respective values of the configuration parameters. The determined configuration parameter values are values of the configuration parameters associated with the template data that corresponds to the identified coil image data.

According to one embodiment, the reference data comprises a coil model of the coil arrangement, wherein comparing the identified data with the reference data comprises fitting the identified data with the coil model. The coil model may for example model the structure, the position and/or orientation of the coil e.g. the position relative to a subject. The model of the coil may for example be obtained using a 3D modeling software and measurements of the 3D geometry of the coil. The measurements may be performed in different position and/or orientation of the coil e.g. with respect to a subject. The configuration parameters values may for example be values that provide the optimal or best fit results of the model to the identified data. This embodiment may enable an accurate recognition method of the coil.

According to one embodiment, the configuration parameter comprises at least one of: the position of the coil arrangement; the orientation of the coil arrangement; the type of the coil arrangement; the routing positioning of the cables of the coil arrangement. The configuration parameters may enable the positioning of the subject as well as the control of the function of the medical apparatus namely the function of the coil arrangement.

According to one embodiment, the coil image data is identified by a machine or deep learning model using the sensor data as input. This embodiment may enable accurate object recognition.

For example, the machine learning model may be generated by executing a learning algorithm on a training set with a known structure of the coil. The term "machine learning" refers to a computer algorithm used to extract useful information from training data by building probabilistic models (referred to as machine learning models) in an automated way. The machine learning may be performed using one or more learning algorithms such as linear regression, K-means, classification algorithm etc. A "model" may for example be an equation or set of rules that makes it possible to predict an unmeasured value (e.g. is the image data corresponds to a coil) from other, known values.

According to one embodiment, the determining of the scan position is performed using the position of the coil, the position of the target volume and the position of a subject support of the subject. According to one embodiment, the scan position may be determined by further using the position of the subject. The scan position may be determined based on the nature of the target volume (e.g. abdomen). For example, in case of an anterior coil, depending whether a cardiac, a liver or a prostate scanning is to be performed, different scan positions are derived from the determined coil position. The scan position may for example be determined by further using an estimated sensitivity profile of the coil.

According to one embodiment, the scanning sensor comprises at least one of a stereoscopic, RGB, NIR, mono, thermography camera, structured light and time of flight range based camera. The use of those cameras may have the following advantages: they allow (e.g. using depth information) extraction of the precise 3-dimensional position and orientation and shape in relation to the coordinate system of the scanning imaging system. This accuracy and repeatability may allow to robustly detect any coil present in the scene even if it is not connected to the system and represent the basis for the coil connection safety.

According to one embodiment, the position of the coil arrangement is relative to a subject support coordinate system, the position of the target volume is relative to a subject coordinate system, the position of the subject is relative to the subject support coordinate system, the position of the subject support is relative a coordinate system of the scanning imaging system, wherein the determination of the scan position comprises registering the positions used for determining the scan position to a same coordinate system.

According to one embodiment, the execution of the instructions further causes the processor to determine the laterality of the scanned field of view relative to the subject coordinate system using the sensor data, wherein the scan position is determined using the determined patient laterality. The scanned field of view is the field of view scanned by the scanning sensor. In another example, the laterality may be determined using ExamCard parameters.

The scanning sensor may be placed in a position that provides a field of view of the camera covering the subject and the coil. For example, the camera maybe mounted on the scanning imaging system. This may be advantageous as there are no additional mounting requirements for placing the camera for example on a ceiling.

According to one embodiment, the identifying of the coil image data comprises identifying an installation failure of the coil arrangement, wherein failure indicates that the coil arrangement is not connected to the scanning imaging system or has a partial connection failure or that a position of the coil arrangement is different from predefined positions; providing a warning of the failure, and repeating the steps upon receiving a confirmation of a fix of the failure. Non connected coils can be signaled to the user so they can be either plugged in if needed or removed from the setup. They would otherwise present a significant safety concern for MR scanning. Also coil cable routing could be checked for safety. In another example, another failure may be identified. The other failure may indicate that the configuration (e.g. routing, lengths and position) of IV infusion tubing or cables is different from a predefined configuration. This may further allow to identify risk of collision of the subject or equipment with the magnet bore when the subject is to be moved into the magnet bore to be imaged.

The execution of the instructions may further cause the processor to adapt a patient model of the subject to the sensor data to determine the patient position. The patient model may comprise parameters indicative of biometric data like height, weight, gender, laterality, anatomical atlas including internal organs. The patient model parameters may be adapted using only the visible surface of the subject. The patient model may be an external surface model with anatomical labels indicating ideal scan position per target or it may contain as well the internal organs that get adapted in 3D along with the outer surface for more precision. The scan position may automatically be computed from the adapted patient model and/or the parameter values.

According to one embodiment, the scanning imaging system is any one of the following: a magnetic resonance imaging system, a combined positron emission tomography and magnetic resonance imaging system, a combined positron emission tomography and computed tomography system, a combined computer tomography and radiation therapy system, a combined computed tomography and positron emission tomography system, and a combined magnetic resonance imaging system and radiation therapy system.

According to one embodiment, the execution of the instructions further causes the processor to determine from the sensor data the position of parts of the coil arrangement relative to a predefined axis of the subject; determine the scan position for a given acquisition with the scanning imaging system relative to a subject support of the subject using other scan positions determined for previously planned acquisitions with the scanning imaging system such that the scan position of the given acquisition is consistent with areas on the subject support covered by the parts of the coil. The previously planned acquisitions may be for scanning the target volume or scanning other target volumes. In other terms, this embodiment enables to determine the position of the parts of the coil arrangement relative to a specific patient axis from the sensor data; the position of the first (planned) MR acquisition relative to the other planned acquisitions, wherein this latter position is known relative to the patient support through the former. And the patient support's first target position is determined by ensuring that the relative positions of the planned scans are consistent with areas on the patient support covered by elements of the coil arrangement. This may enable relative positioning of multiple target volumes, corresponding with multiple parts of the coil arrangement.

In another aspect, the invention relates to a method for imaging a target volume of a subject by a medical apparatus. The medical apparatus comprises a coil arrangement being configured to be disposed on the subject and to receive signals from the subject; a scanning sensor; and a scanning imaging system being configured for acquiring image data from a predefined imaging zone using the coil. The method comprises:
- acquiring with the scanning sensor, sensor data that can be used to generate position attributes of the subject and the coil arrangement;
- identifying coil image data representing the coil arrangement in the sensor data;
- determining values of one or more configuration parameters of the coil arrangement using the identified coil image data;
- determining using the parameter values and the sensor data a scan position indicative of a position of the target volume within the imaging zone; and
- controlling the scanning imaging system for acquiring the image data from the target volume in accordance with the determined scan position.

In another aspect, the invention relates to a computer program product comprising machine executable instructions for execution by a processor, wherein execution of the machine executable instructions causes the processor to perform the method of previous embodiment.

For example, in a MRI system a monitoring unit may be provided with depth camera (s) to acquire 3D image information of a patient and surface coils placed on the patient's body. The monitoring unit with an image processing unit is configured to recognize surface coil type and position and patient position from the 3D image. The volumetric position and recognition allows to optimize the scan region from which MR signal are acquired to the 3D coil sensitivity profile. The image processing unit may also be configured to identify surface coils that are not properly connected to the MRI system, or to identify positional problems (such as collisions, cabling or tubing getting stuck).

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

A medical imaging system as used herein is a medical device configured for acquiring medical image data from an imaging volume. Medical image data is data which is descriptive of a volume of a subject within the imaging volume. Medical image data may be reconstructed into or rendered as one or more medical images.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical image data. A Magnetic Resonance (MR) image or magnetic resonance image data is defined herein as being the reconstructed two or three dimensional visualization of anatomic data contained within the magnetic resonance data.

### BRIEF DESCRIPTION OF THE DRAWINGS

In the following preferred embodiments of the invention will be described, by way of example only, and with reference to the drawings in which:
Fig. 1 illustrates an example of a medical apparatus;
Fig. 2 shows a flowchart which illustrates an example of a method of operating the medical apparatus of Fig. 1.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

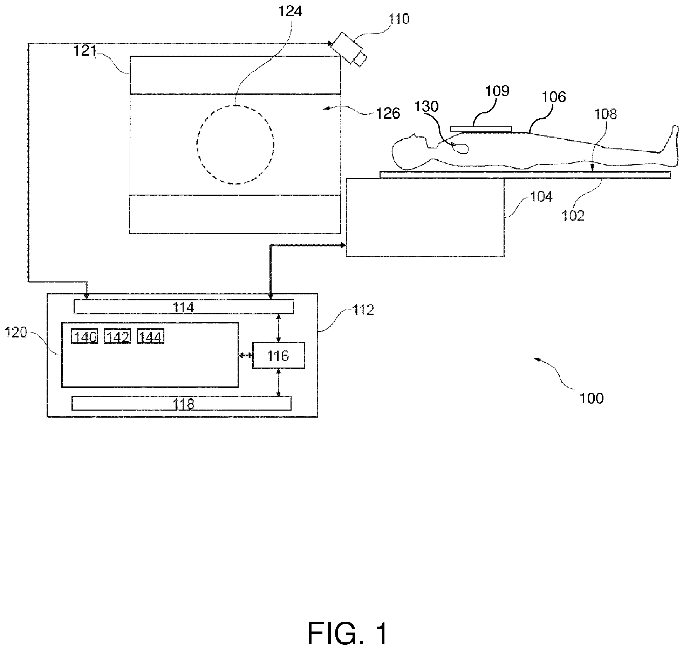

Fig. 1 illustrates an example of a medical apparatus 100. The medical apparatus 100 is shown as comprising a scanning imaging system 121. The scanning imaging system 121 has a medical imaging zone or volume 124 from which medical imaging data can be received or acquired. The medical apparatus 100 further comprises a subject support 102. The subject support 102 comprises an actuator 104 which is constructed to move the subject support 102 a controlled distance or displacement e.g. along a linear path. There is a subject 106 reposing on a support surface 108 of the subject support 102. The subject support 102 is configured for moving at least a portion of the subject 106 into the medical imaging zone 124. In this example the scanning imaging system 302 is cylindrical and has a bore 126 which the subject 106 can be moved into using the subject support 102. This is however not necessary, not all scanning imaging systems 121 need to be cylindrically symmetric as is illustrated in Fig. 1.

A coil arrangement of one or more local coils may be arranged on the subject 106. For exemplification purpose, only one coil 109 is shown as being arranged on the abdomen of the subject 106; however more coils can be arranged such as a head coil etc. The coil 109 is configured to receive radio frequency signals coming out of the subject 106 and in particular from a target volume 130 of the subject.

The support surface 108 is facing a camera 110. The camera 110 is configured to acquire 3D image data or sensor data of the subject including the coil 109 when the subject support 102 is in a predefined preparation position before being placed in the scanning imaging system 121 (e.g. for coil positioning during preparation for magnetic resonance scanning). For example, the camera 110 is positioned such that it surveilles the examination preparation and the subject. The camera may be an intensity- or depth-sensing camera allowing to derive a 3D representation of the subject and objects. The 3D representation may have the following advantages: it allows extraction of the precise 3-dimensional position and orientation and shape in relation to the coordinate system of the scanning imaging system. This accuracy and repeatability also allows to robustly detect any coil present in the scene even if it is not connected to the system and represents the basis for the coil connection safety.

The medical apparatus 100 is further shown as comprising a computer 112. The computer 112 comprises a hardware interface 114 that enables a processor 116 to communicate with and control the other components of the medical apparatus 100.

Specifically in Fig. 1 the hardware interface 114 is shown as interfacing with the camera 110 to acquire 3D image data and with the actuator 104 to control the position of the subject support 102. In other examples the hardware interface 114 may be used to control additional components.

The processor 116 is in communication with the hardware interface 114, a memory 120, and a user interface 118. The memory 120 may be any combination of memory which is accessible to the processor 116. This may include such things as main memory, cached memory, and also non-volatile memory such as flash RAM, hard drives, or other storage devices. In some examples the memory 120 maybe considered to be a non-transitory computer-readable medium.

The memory 120 is shown as containing machine-executable instructions 140. The machine-executable instructions 140 enable the processor 116 to control the operation and function of the medical apparatus 100. The memory 120 is further shown as containing a 3D image data 142 that was acquired by the camera 110 when the subject support 102 was in the preparation position.

The memory 120 is further shown as containing a reference data 144 descriptive of the coil 109. For example, the reference data 104 may comprise a 3D coil model of the coil 109. The 3D coil model may for example be a volume or surface model which is deformed or fit to at least part of the 3D image data. The 3D coil model may for example be used to derive information descriptive of the coil, such things as an estimate of the subject size, height, and/or weight.

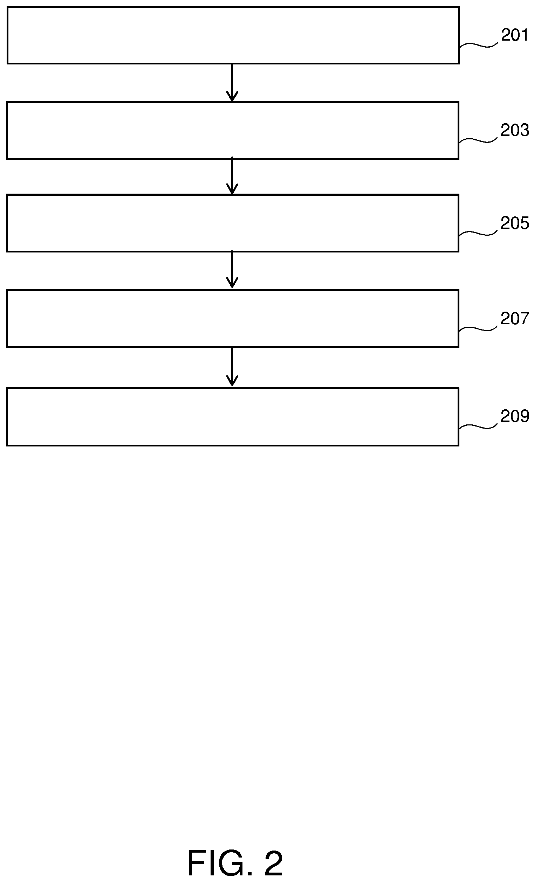

Fig. 2 is a flowchart which illustrates an example of a method of operating the medical apparatus of Figure 1 for imaging a target volume 130 of a subject 106. In this example of Figure 2, the coil arrangement of the medical apparatus comprises one coil.

In step 201, 3D image data (also referred to as sensor data or volumetric image information) may be acquired with the camera 110. The 3D image data may be used to generate a 3D representation indicative of the coil 109 and/or the subject 106. The 3D image data may be obtained from one or more video sequences. The camera 110 may be configured to shoot the coil and subject at different angles.

In step 203, coil image data representing the coil in the 3D image data maybe identified. For example, using image processing and object recognition techniques such as machine learning, template matching, or feature detection combined with modelling or barcode identification techniques, the coil 109 may be detected as soon as it becomes detectable in the camera surveillance scene. Step 203 may for example be performed using a predefined object recognition algorithm. Approaches based on machine learning or computer vision may be employed for robust coil recognition.

In step 205, values of one or more configuration parameters of the coil may be determined using the identified coil image data. The configuration parameter may comprise the position and/or orientation of the coil 109. For example, upon detecting by the object recognition algorithm the presence and coarse location of the coil 109, the exact position and orientation can be determined, e.g. a respective 3D coil model can be fitted to the 3D image data that represents the coil. The coil position and the coil orientation may be along the longitudinal direction of the subject support 102.

In step 207, the parameter values may be used to determine a scan position indicative of a position of the target volume 130 within the imaging zone 124. The scan position or scan region may be derived in 3D from a sensitivity profile of the coil 109, from the position of the target volume, the calculated position of the coil 109, the position of the subject 106, the position of the subject support 102. For determining the above positions, the subject support may be referenced to the magnet or bore 126, the subject may be referenced to the subject support 102, the coil 109 maybe referenced to the subject support 102 and the target volume may be referenced to the subject 106. The determination of the scan position may comprise the registration or transformation of the different positions into one coordinate system e.g. the coordinate system of the medical apparatus 100.

The scan position and/or coil position and orientation may be determined in a predefined coordinate system e.g. the coordinate system of the medical apparatus 100.

In step 209, the scanning imaging system 121 may be controlled for imaging the target volume 130 in accordance with the determined scan position. For example, the subject support 102 may be moved such that the scan position is reached.

In one example, step 203 may further comprise identifying an installation failure of the coil 109, wherein failure indicates that the coil 109 is not connected to the scanning imaging system 121 or that positions of the coil are different from predefined positions. In this case, a warning of the failure may be provided e.g. to the user of the scanning imaging system 121 and upon receiving a confirmation of a fix of the failure (e.g. from the user) the method steps 201-209 may be executed.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical apparatus
- 102: subject support
- 104: actuator
- 106: subject
- 108: support surface
- 110: camera
- 112: computer
- 114: hardware interface
- 116: processor
- 118: user interface
- 120: memory
- 121: scanning imaging system
- 124: imaging zone
- 126: bore
- 130: target volume
- 140: machine executable instructions
- 142: 3D image data
- 144: reference data
- 201-209: method steps.

## Claims

1. A medical apparatus (100) for imaging a target volume (130) of a subject (106), comprising:
- a coil arrangement (109) being configured to be disposed on the subject (106) and to receive signals from the subject;
- a scanning sensor (110);
- a scanning imaging system (121) being configured for acquiring image data from a predefined imaging zone (124) using the coil arrangement (109);
- a memory (120) containing machine executable instructions (140);
- a processor (116), wherein execution of the machine executable instructions (140) cause the processor (116) to:
- acquire with the scanning sensor (110) sensor data that can be used to generate position attributes of the subject (106) and the coil arrangement;
- identify coil image data representing the coil arrangement (109) in the sensor data (142);
- determine values of one or more configuration parameters of the coil arrangement (109) using the identified coil image data;
- determine using the parameter values and the sensor data (142) a scan position indicative of a position of the target volume (130) within the imaging zone (124); and
- control the scanning imaging system (121) for acquiring the image data from the target volume (130) in accordance with the determined scan position.

2. The medical apparatus of claim 1, wherein the position attributes of the subject and coil arrangement define a three-dimensional, 3D, representation of the subject and the coil arrangement.

3. The medical apparatus of claim 1 or 2, wherein the coil image data is identified by a machine or deep learning model using the sensor data (142) as input.

4. The medical apparatus of claim 1 or 2 or 3, wherein the determining of the configuration parameters values comprises comparing the identified data with reference data (144) of the coil arrangement (109).

5. The medical apparatus of claim 4, wherein the reference data (144) comprises a coil model of the coil arrangement (109), wherein comparing the identified data with the reference data (144) comprises fitting the identified data with the coil model.

6. The medical apparatus of any of the preceding claims, wherein the configuration parameter comprises at least one of: the position of the coil arrangement; the orientation of the coil arrangement; the type of the coil arrangement; the routing positioning of the cables of the coil arrangement.

7. The medical apparatus of any of the preceding claims, wherein the determining of the scan position is performed using the position of the coil, the position of a subject support of the subject, and the position of the target volume (130).

8. The medical apparatus of claim 7, wherein the position of the coil arrangement is relative to a subject support coordinate system, the position of the target volume is relative to a subject coordinate system, the position of the subject is relative to the subject support coordinate system, the position of the subject support is relative to a coordinate system of the scanning imaging system, wherein the determination of the scan position comprises registering or transforming the positions used for determining the scan position into a same coordinate system.

9. The medical apparatus of claim 8, wherein the execution of the instructions further causes the processor to determine the laterality of the scanned field of view relative to the subject coordinate system using the sensor data, wherein the scan position is determined using the determined patient laterality.

10. The medical apparatus of any of the preceding claims, wherein the execution of the instructions further causes the processor to determine from the sensor data the position of parts of the coil arrangement relative to a predefined axis of the subject; determine the scan position for a given acquisition with the scanning imaging system relative to a subject support of the subject using other scan positions determined for previously planned acquisitions with the scanning imaging system such that the scan position of the given acquisition is consistent with areas on the subject support covered by the parts of the coil arrangement.

11. The medical apparatus of any of the preceding claims, wherein the scanning sensor (109) is mounted at the scanning imaging system to enable a complete survey of the examination preparation workflow scenery (121).

12. The medical apparatus of any of the preceding claims, wherein the identifying of the coil image data comprises
identifying an installation failure of the coil arrangement (109), wherein failure indicates that the coil arrangement (109) is not connected to the scanning imaging system or has a partial connection failure or that positions of the coil arrangement are different from predefined positions;
providing a warning of the failure, and guidance to resolve the failure and determining that the failure is eliminated.

13. The medical apparatus of any one of claims, wherein the scanning imaging system (121) is any one of the following: a magnetic resonance imaging system, a combined positron emission tomography and magnetic resonance imaging system, a combined positron emission tomography and computed tomography system, a combined computer tomography and radiation therapy system, a combined computed tomography and positron emission tomography system, and a combined magnetic resonance imaging system and radiation therapy system.

14. A method for imaging a target volume (130) of a subject (106) by a medical apparatus (100) comprising a coil arrangement (109) being configured to be disposed on the subject (106) and to receive signals from the subject (106); a scanning sensor (110); a scanning imaging system (121) being configured for acquiring image data from a predefined imaging zone (124) using the coil arrangement (109); the method comprising:
- acquiring (201) with the scanning sensor (110) sensor data that can be used to generate position attributes position attributes of the subject and the coil arrangement;
- identifying (203) coil image data representing the coil arrangement in the sensor data (142);
- determining (205) values of one or more configuration parameters of the coil arrangement (109) using the identified coil image data;
- determining (207) using the parameter values and the sensor data (142) a scan position indicative of a position of the target volume within the imaging zone (124); and
- controlling (209) the scanning imaging system (121) for acquiring the image data from the target volume (130) in accordance with the determined scan position.

15. A computer program product comprising machine executable instructions for execution by a processor, wherein execution of the machine executable instructions causes the processor to perform the method of claim 14.
